# EUROPÄISCHE PATENTANMELDUNG

(11) **EP 1 223 153 A1**
(43) Veröffentlichungstag der Anmeldung: **17.07.2002**
(21) Anmeldenummer: 02008233.5
(22) Anmeldetag: 01.04.1999
(51) Int. Cl.: C07C 29/141

(54) **Verfahren zur Herstellung von Zuckeralkoholen unter Verwendung des In-situ -Katalysators**

(30) Priorität: 07.04.1998 DE 19815639
(62) Teilanmeldung aus: 99106109.4
(71) Anmelder: BASF AKTIENGESELLSCHAFT, 67056 Ludwigshafen (DE)
(72) Erfinder: Böttcher, Arnd, Dr., 67227 Frankenthal (DE); Breitscheidel, Boris, Dr., 67117 Limburgerhof (DE); Henkelmann, Jochem, Dr., 68165 Mannheim (DE); Brunner, Melanie, Dr., 67105 Schifferstadt (DE)
(74) Vertreter: Isenbruck, Günter, Dr.

(57) **Zusammenfassung**

Inkontaktbringen des Zuckers oder des Gemisches aus zwei oder mehr davon mit Wasserstoff in Gegenwart eines Katalysators, wobei ein Zuckeralkohol oder ein Gemisch aus zwei oder mehr davon erhalten wird, dadurch gekennzeichnet, dass der Katalysator mindestens eine in situ auf einem Träger abgeschiedene, homogene Verbindung mindestens eines Metalls der VIII. Nebengruppe des Periodensystems alleine oder zusammen mit mindestens einem Metall der I. oder VII. Nebengruppe des Periodensystems umfasst.

## Beschreibung

Die vorliegende Erfindung betrifft ein Verfahren zur Herstellung von Zuckeralkoholen durch Hydrierung von Zuckern unter Verwendung von "In-situ"-Katalysatoren, die ein oder mehrere Metalle der VIII. Nebengruppe des Periodensystems als Aktivkomponente(n) umfassen.

Zuckeralkohole, wie z.B. Sorbitol, Mannitol, Maltitol und Xylitol finden breite Anwendung in der Lebensmittelindustrie, der Kosmetik, der Pharmazie und auf dem technischen Sektor.

Verfahren zur Herstellung von Zuckeralkoholen aus den korrespondierenden Zuckern durch Hydrierung, insbesondere diskontinuierliche Verfahren, bei denen pulverförmige Metall-Katalysatoren, wie z.B. Nickel-Katalysatoren, in einem Suspensionsverfahren zum Einsatz kommen, sind aus dem Stand der Technik bekannt (s. Ullmanns Encykl. der Technischen Chemie, Bd. 24, S. 772 (1983)).

Die EP-A 0 773 063 beschreibt ein kontinuierliches Verfahren zur Hydrierung von Zuckern an einem Raney-Ni-Al-Kontakt bei 130°C und 150 bar Druck.

Hydrierungen von Zuckern an geträgerten Ru-Kontakten sind ebenfalls beschrieben. Die US 4 471 144 beschreibt die Hydrierung von Kohlehydraten in wässriger Lösung in Gegenwart eines Ruthenium auf θ-Al₂O₃-Katalysators. Die US 4 487 980 beschreibt ein ebensolches Verfahren in dem ein Katalysator mit einem Metall der VIII. Nebengruppe und TiO₂ als Träger eingesetzt wird. Die US 4 380 680 beschreibt die Verwendung eines Trägerkatalysators mit α-Al₂O₃ als Träger und einem Metall ausgewählt unter Os, Ru, Pd und Pt als Aktivkomponente bei der Hydrierung von Zuckern unter Erhalt von Zuckeralkoholen.

Eine Untersuchung bzgl. der bei derartigen Hydrierungen auftretenden Deaktivierung der verwendeten Katalysatoren am Beispiel der Hydrierung von Glucose unter Verwendung von Ru auf Al₂O₃ als Katalysator ist in einem wissenschaftlichen Artikel in Applied Catalysis A: General 87 (1992), S. 219-229 beschrieben.

Obwohl, wie sich aus obiger Zusammenfassung des Standes der Technik ergibt, bereits einige Verfahren zur Hydrierung von Zuckern bekannt sind, weisen die bislang verwendeten Katalysatoren nicht selten kurze Standzeiten auf, die durch eine Deaktivierung oder ein "Ausbluten" des Katalysators bedingt sind. Ferner kommt es während der Hydrierung häufig zu einer merklichen Epimerisierung, Zersetzung oder Polymerisation der Zuckeralkohole unter den gewählten Bedingungen.

Demgemäß besteht eine Aufgabe der vorliegenden Erfindung darin, neue Verfahren zur Hydrierung von Zuckern bereit zu stellen, in denen spezielle Katalysatoren mit einem oder mehreren Metallen der VIII. Nebengruppe des Periodensystems als Aktivmetall eingesetzt werden. Mit diesen neuen Verfahren soll es insbesondere ermöglicht werden, nahezu Epimeren-freie Zuckeralkohole in sehr hohen Ausbeuten bei nahezu vollständigem Umsatz zu erhalten. Darüber hinaus soll gegenüber den herkömmlichen Verfahren lediglich ein minimaler Anteil an Nebenprodukten bzw. Zersetzungsprodukten während der Hydrierung anfallen, um eine nachfolgende Aufarbeitung der Zuckeralkohole in einfacher und ökonomischer Weise durchführen zu können.

Demgemäß betrifft die vorliegende Erfindung in einer Ausführungsform ein Verfahren zur Hydrierung eines Zuckers oder eines Gemisches aus zwei oder mehr davon, das die folgende Stufe umfasst:
Inkontaktbringen des Zuckers oder des Gemisches aus zwei oder mehr davon mit Wasserstoff in Gegenwart eines Katalysators, wobei ein Zuckeralkohol oder ein Gemisch aus zwei oder mehr davon erhalten wird, dadurch gekennzeichnet, dass der Katalysator mindestens eine in situ auf einem Träger abgeschiedene, homogene Verbindung mindestens eines Metalls der VIII. Nebengruppe des Periodensystems alleine oder zusammen mit mindestens einem Metall der I. oder VII. Nebengruppe des Periodensystems umfasst (Katalysator 1).

In einer weiteren Ausführungsform betrifft die vorliegende Erfindung ein Verfahren zur Hydrierung eines Zuckers oder eines Gemisches aus zwei oder mehr davon, das die folgende Stufe umfasst:
Inkontaktbringen des Zuckers oder des Gemisches aus zwei oder mehr davon mit Wasserstoff in Gegenwart eines Katalysators, wobei ein Zuckeralkohol oder ein Gemisch aus zwei oder mehr davon erhalten wird, dadurch gekennzeichnet, dass der Katalysator als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfasst, wobei 5 bis 50% des Porenvolumens des Trägers von Makroporen mit einem Porendurchmesser im Bereich von 50 nm bis 10.000 nm und 50 bis 95% des Porenvolumens des Trägers von Mesoporen mit einem Porendurchmesser im Bereich von 2 bis 50 nm gebildet werden, wobei sich die Summe der Porenvolumina zu 100% addiert (Katalysator 2).

Ferner betrifft sie ein Verfahren zur Hydrierung eines Zuckers oder eines Gemisches aus zwei oder mehr davon, das die folgende Stufe umfasst:
Inkontaktbringen des Zuckers oder des Gemisches aus zwei oder mehr davon mit Wasserstoff in Gegenwart eines Katalysators, wobei ein Zuckeralkohol oder ein Gemisch aus zwei oder mehr davon erhalten wird, dadurch gekennzeichnet, dass der Katalysator ein monolithischer Trägerkatalysator ist, der durch aufeinanderfolgendes an der Luft Erhitzen und Abkühlen eines Trägermaterials in Form eines Metallgewebes oder einer Metallfolie, nachfolgendes Beschichten im Vakuum mit einer Aktivkomponente und anschließendes Schneiden und Verformen des beschichteten Trägermaterials und abschließendes Verarbeiten zu einem monolithischen Trägerkatalysator hergestellt werden kann, wobei als Aktivmetall mindestens ein Metall der VIII. Nebengruppe des Periodensystems alleine oder zusammen mit mindestens einem Metall der I. oder VII. Nebengruppe des Periodensystems verwendet wird (Katalysator 3).

Als Aktivmetall können prinzipiell alle Metalle der VIII. Nebengruppe des Periodensystems eingesetzt werden. Vorzugsweise werden als Aktivmetalle Platin, Rhodium, Palladium, Cobalt, Nickel oder Ruthenium oder ein Gemisch aus zwei oder mehr davon eingesetzt, wobei insbesondere Ruthenium als Aktivmetall verwendet wird. Unter den ebenfalls verwendbaren Metallen der I. oder VII. oder aber der I. und der VII. Nebengruppe des Periodensystems, die ebenfalls allesamt prinzipiell verwendbar sind, werden vorzugsweise Kupfer und/oder Rhenium eingesetzt.

Die Begriffe "Makroporen" und "Mesoporen" werden im Rahmen der vorliegenden Erfindung so verwendet, wie sie in Pure Appl. Chem., 45, S. 79 (1976) definiert sind, nämlich als Poren, deren Durchmesser oberhalb von 50 nm (Makroporen) oder deren Durchmesser zwischen 2 nm und 50 nm liegt (Mesoporen).

Der Gehalt des Aktivmetalls beträgt im allgemeinen ungefähr 0,01 bis ungefähr 30 Gew.-%, vorzugsweise ungefähr 0,01 bis ungefähr 5 Gew.-% und insbesondere ungefähr 0,1 bis ungefähr 5 Gew.-%, jeweils bezogen auf das Gesamtgewicht des verwendeten Katalysators, wobei die bei den Katalysatoren 1 bis 3 vorzugsweise verwendeten Gehalte nochmals bei der Diskussion dieser Katalysatoren einzeln angegeben sind.

Sofern zur Herstellung der erfmdungsgemäßen Katalysatoren übliche Katalysatorträgersysteme, wie z.B. Aktivkohle, Siliciumcarbid, Aluminiumoxid, Siliciumdioxid, Titandioxid, Zirconiumdioxid, Magnesiumoxid, Zinkoxid oder deren Gemische, eingesetzt werden, werden diese jeweils in Kugel-, Strang- oder Ringform, sofern sie als Festbettkontakt eingesetzt werden, sowie als Split, bzw. feinkörniges Granulat für die Verwendung in Suspension, verwendet. Weitere Details bzgl. dieser Trägersysteme sind der Diskussion bei den jeweiligen Katalysatoren 1 bis 3 zu entnehmen.

Im Rahmen der vorliegenden Verfahren können prinzipiell alle Zucker eingesetzt werden. Dabei umfasst der erfindungsgemäß verwendete Begriff "Zucker" sowohl Monosaccharide, wie z.B. Glucose, Mannose, Galactose, Talose, Fructose, Allose, Altrose, Idose, Gulose, Xylose, Ribose, Arabinose, Lyxsose, Threose und Erythrose, Di- und Trisaccharide wie z.B. Maltose, Lactose, Cellobiose, Sucrose, Melibiose und Raffinose, und Polysaccharide wie z.B. Stärke, Stärkezersetzungsprodukte, Cellulose und Cellulosezersetzungsprodukte, wie z.B. Dextrin, Glucosesirup, Cellulosehydrolysate und Stärkehydrolysate, wie z.B. Maisstärkehydrolysate.

Vorzugsweise werden im Rahmen der erfindungsgemäßen Verfahren Glucose zu Sorbitol, Mannose zu Mannitol, Fructose zu einer Mischung aus Sorbitol und Mannitol, Xylose zu Xylitol, Lactose zu Lactitol und Maltose zu Maltitol umgesetzt.

### KATALYSATOR 1

Das erfindungsgemäße Verfahren kann in Gegenwart eines Katalysators 1, der mindestens eine in situ auf einem Träger abgeschiedene, homogene Verbindung mindestens eines Metalls der VIII. Nebengruppe des Periodensystems, ggf. zusammen mit mindestens einer homogenen Verbindung mindestens eines Metalls der I. oder VII. Nebengruppe des Periodensystems, umfasst, durchgeführt werden. Die Herstellung der Katalysatoren erfolgt dadurch, dass eine homogene Metallverbindung während der Reaktion mit dem Eintrag in den Reaktor eingebracht wird und sich während der Reaktion auf einen im Reaktor befindlichen Träger abscheidet.

Ferner kann die homogene Metallverbindung auch vor der Umsetzung in den Reaktor eingebracht werden und sich während einer Behandlung mit Wasserstoff auf einen im Reaktor befindlichen Träger abscheiden.

Der im Rahmen der vorliegenden Anmeldung verwendete Begriff "in situ" bedeutet, dass der Katalysator nicht separat hergestellt und getrocknet wird und dann als sozusagen fertiger Katalysator in den Reaktor eingebracht wird, sondern dass der Katalysator im Rahmen der vorliegenden Erfindung im Reaktor entweder unmittelbar vor oder während der eigentlichen Hydrierung gebildet wird.

Der im Rahmen der vorliegenden Anmeldung verwendete Begriff "homogene Verbindung eines Metalls der VIII., I. oder VII. Nebengruppe des Periodensystems" bzw. "homogene Rutheniumverbindung" bedeutet, dass die erfindungsgemäß verwendete Metallverbindung in dem sie umgebenden Medium, d.h. des eingesetzten, noch zu hydrierenden Zuckers der in Form einer wässrigen Lösung vorliegt, löslich ist.

Als Metallverbindungen kommen dabei vor allem Nitrosylnitrate und Nitrate, aber auch Halogenide, Carbonate, Carboxylate, Acetylacetonate, Chlor-, Nitrido- und Aminkomplexe sowie Oxidhydrate oder deren Gemische zum Einsatz. Bevorzugte Verbindungen sind Rutheniumnitrosylnitrat, Ruthenium(III)chlorid, Ruthenium(III)nitrat und Rutheniumoxidhydrat.

Obwohl die Menge der im Rahmen des erfindungsgemäßen Verfahrens auf den oder die Träger aufgebrachten Metallverbindung nicht in besonderer Weise beschränkt ist, wird unter den Gesichtspunkten einer ausreichenden katalytischen Aktivität und der Wirtschaftlichkeit des Verfahrens das Metallsalz oder der Metallkomplex in einer solchen Mengen auf den oder die Träger aufgebracht, dass sich 0,01 bis 30 Gew.-%, bezogen auf das Gesamtgewicht des Katalysators, an Aktivmetall auf dem oder den Trägern abscheidet. Weiter bevorzugt beträgt diese Menge 0,2 bis 15 Gew.-%, insbesondere bevorzugt etwa 0,5 Gew.-%.

Die im Reaktor befindlichen Träger sind vorzugsweise Metallnetze und Metallringe sowie Steatitkörper, wie sie unter anderem in der EP-A-0 564 830 und der EP-A-0 198 435 beschrieben werden. Im folgenden sollen dennoch kurz die im Rahmen der vorliegenden Erfindung besonders bevorzugt verwendeten Träger und deren Herstellung erläutert werden.

Besonders bevorzugt werden metallische Trägermaterialien, wie beispielsweise die Edelstähle mit Werkstoffnummern 1.4767, 1.4401, 2.4610, 1.4765, 1.4847, 1.4301 usw. verwendet, da sie vor der Beschichtung mit den Aktivkomponenten durch eine Temperung eine Aufrauung ihrer Oberfläche erhalten können. Insbesondere bevorzugt werden als Netzmaterial Kanthal (Werkstoffnr. 1.4767) bzw. Metalle, die Aluminium enthalten, eingesetzt. Kanthal ist eine Legierung, die zu ungefähr 75 Gew.-% Fe, ungefähr 20 Gew.-% Cr und ungefähr 5 Gew.-% Al enthält. Für die Temperung werden die oben erwähnten metallischen Träger bei Temperaturen von 600 bis 1100, vorzugsweise 800 bis 1000 °C, eine bis zwanzig, vorzugsweise eine bis zehn, Stunden an der Luft erhitzt und wieder abgekühlt. Diese Vorbehandlung ist für die Aktivität des Katalysators entscheidend, da ohne diese Temperungsbehandlung praktisch kein Ruthenium in situ auf den metallischen Träger abgeschieden werden kann. Nach dieser Trägerbehandlung bei erhöhter Temperatur werden diese mit der Rutheniumverbindung beschichtet.

In einer weiter bevorzugten Ausführungsform können die oben beschriebenen Träger mit einer Schicht eines Palladiummetalls, wie z.B. Ni, Pd, Pt, Rh, vorzugsweise Pd, in einer Dicke von ungefähr 0,5 bis ungefähr 10 nm, insbesondere circa 5 nm, bedampft werden, wie dies ebenfalls in der bereits oben erwähnten EP-A-0 564 830 beschrieben wird.

Im Rahmen der vorliegenden Erfindung wird als Katalysator insbesondere ein Netz aus getempertem Kanthal, auf das eine Pd-Schicht zur Erleichterung der Abscheidung des Aktivmetalls mit einer Dicke von ungefähr 5 nm aufgedampft wurde, als Träger verwendet.

Es können jedoch auch übliche Katalysatorträgersysteme, wie zum Beispiel Aktivkohle, Siliciumcarbid, Aluminiumoxid, Siliciumdioxid, Titandioxid, Zirconiumdioxid, Magnesiumoxid, Zinkoxid oder deren Gemische, jeweils in Kugel-, Strang- oder Ringform, sowie als Split, bzw. feinkörniges Granulat eingesetzt werden. Unter diesen besonders bevorzugt sind Aluminiumoxid und Zirconiumdioxid. Dabei ist die Porengröße sowie die Porenverteilung völlig unkritisch. Es können bimodale und auch alle anderen Arten von Trägern eingesetzt werden. Vorzugsweise sind die Träger makroporös.

Weitere Details bezüglich des Katalysators 1 bzw. zu seiner Herstellung sind der DE-A 196 22 705.4 zu entnehmen, deren diesbezüglicher Inhalt durch Bezugnahme vollständig in die vorliegende Anmeldung einbezogen wird.

### KATALYSATOR 2

Die erfindungsgemäß verwendeten Katalysatoren 2 enthalten ein oder mehrere Metalle der VIII. Nebengruppe des Periodensystems als Aktivkomponente(n) auf einem Träger, wie hierin definiert. Bevorzugt werden Ruthenium, Palladium und/oder Rhodium als Aktivkomponente(n) verwendet.

Die erfindungsgemäß verwendeten Katalysatoren 2 können technisch hergestellt werden durch Auftragen mindestens eines Aktivmetalls der VIII. Nebengruppe des Periodensystems, vorzugsweise Ruthenium oder Palladium und gegebenenfalls mindestens einem Metall der I. oder VII. Nebengruppe des Periodensystems auf einem geeigneten Träger. Die Auftragung kann durch Tränken des Trägers in wässrigen Metallsalzlösungen, wie z.B. Ruthenium- oder Palladiumsalzlösungen, durch Aufsprühen entsprechender Metallsalzlösungen auf den Träger oder durch andere geeignete Verfahren erreicht werden. Als Metallsalze zur Herstellung der Metallsalzlösungen eignen sich die Nitrate, Nitrosylnitrate, Halogenide, Carbonate, Carboxylate, Acetylacetonate, Chlorkomplexe, Nitritokomplexe oder Aminkomplexe der entsprechenden Metalle, wobei die Nitrate und Nitrosylnitrate bevorzugt sind.

Bei Katalysatoren, die mehrere Aktivmetalle auf den Träger aufgetragen enthalten, können die Metallsalze bzw. Metallsalzlösungen gleichzeitig oder nacheinander aufgebracht werden.

Die mit der Metallsalzlösung beschichteten bzw. getränkten Träger werden anschließend getrocknet, wobei Temperaturen zwischen 100°C und 150°C bevorzugt sind. Wahlweise können diese Träger bei Temperaturen zwischen 200°C und 600°C, vorzugsweise 350°C bis 450°C calciniert werden. Anschließend werden die beschichteten Träger durch Behandlung in einem Gasstrom, der freien Wasserstoff enthält, bei Temperaturen zwischen 30°C und 600°C, vorzugsweise zwischen 100°C und 450°C und insbesondere 100°C bis 300°C aktiviert. Der Gasstrom besteht vorzugsweise aus 50 bis 100 Vol.-% H₂ und 0 bis 50 Vol.-% N₂.

Werden auf die Träger mehrere Aktivmetalle aufgetragen und erfolgt das Auftragen nacheinander, so kann der Träger nach jedem Auftragen bzw. Tränken bei Temperaturen zwischen 100°C und 150°C getrocknet werden und wahlweise bei Temperaturen zwischen 200°C und 600°C calciniert werden. Dabei kann die Reihenfolge, in der die Metallsalzlösung aufgetragen oder aufgetränkt werden, beliebig gewählt werden.

Die Metallsalzlösung wird in einer solchen Menge auf den/die Träger aufgebracht, dass der Gehalt an Aktivmetall 0,01 bis 30 Gew.-%, vorzugsweise 0,01 bis 10 Gew.-%, weiter bevorzugt 0,01 bis 5 Gew.-%, und insbesondere 0,3 bis 1 Gew.-% bezogen auf das Gesamtgewicht des Katalysators, beträgt.

Die Metalloberfläche auf dem Katalysator beträgt insgesamt vorzugsweise 0,01 bis 10 m²/g, besonders bevorzugt 0,05 bis 5 m²/g und weiter bevorzugt 0,05 bis 3 m²/g des Katalysators. Die Metalloberfläche wurde durch das Chemisorptionsverfahren gemessen, wie es in J. LeMaitre et al., *"Characterization of Heterologous Catalysts",* Hrsg. Francis Delanney, Marcel Dekker, New York (1984), S. 310- 324, beschrieben ist.

Im erfindungsgemäß verwendeten Katalysator 2 beträgt das Verhältnis der Oberflächen des mindestens einen Aktivmetalls und des Katalysatorträgers weniger als ungefähr 0,3, vorzugsweise weniger als ungefähr 0,1 und insbesondere ungefähr 0,05 oder weniger, wobei der untere Grenzwert bei ungefähr 0,0005 liegt.

Die zur Herstellung der erfindungsgemäß verwendeten Katalysatoren 2 verwendbaren Trägermaterialien besitzen Makroporen und Mesoporen.

Dabei weisen die erfindungsgemäß verwendbaren Träger eine Porenverteilung auf, der gemäß ungefähr 5 bis ungefähr 50%, vorzugsweise ungefähr 10 bis ungefähr 45%, weiter bevorzugt ungefähr 10 bis ungefähr 30 und insbesondere ungefähr 15 bis ungefähr 25% des Porenvolumens von Makroporen mit Porendurchmesser im Bereich von ungefähr 50 nm bis ungefähr 10.000 nm und ungefähr 50 bis ungefähr 95%, vorzugsweise ungefähr 55 bis ungefähr 90%, weiter bevorzugt ungefähr 70 bis ungefähr 90% und insbesondere ungefähr 75 bis ungefähr 95% des Porenvolumens von Mesoporen mit einem Porendurchmesser von ungefähr 2 bis ungefähr 50 nm gebildet werden, wobei sich jeweils die Summe der Porenvolumina zu 100% addiert.

Das Gesamtporenvolumen der erfindungsgemäß verwendeten Träger beträgt ungefähr 0,05 bis 1,5 cm³/g, vorzugsweise 0,1 bis 1,2 cm³/g und insbesondere ungefähr 0,3 bis 1,0 cm³/g. Der mittlere Porendurchmesser der erfindungsgemäß verwendeten Träger beträgt ungefähr 5 bis 20 nm, vorzugsweise ungefähr 8 bis ungefähr 15 nm und insbesondere ungefähr 9 bis ungefähr 12 nm.

Vorzugsweise beträgt die Oberfläche des Trägers ungefähr 50 bis ungefähr 500 m²/g, weiter bevorzugt ungefähr 200 bis ungefähr 350 m²/g und insbesondere ungefähr 250 bis ungefähr 300 m²/g des Trägers.

Die Oberfläche des Trägers wird nach dem BET-Verfahren durch N₂-Adsorption, insbesondere nach DIN 66131, bestimmt. Die Bestimmung des mittleren Porendurchmesser und der Größenverteilung erfolgt durch Hg-Porosimetrie, insbesondere nach DIN 66133.

Obwohl prinzipiell alle bei der Katalysatorherstellung bekannten Trägermaterialien, d.h. die die oben definierte Porengrößenverteilung aufweisen, eingesetzt werden können, werden vorzugsweise Aktivkohle, Siliciumcarbid, Aluminiumoxid, Siliciumdioxid, Titandioxid, Zirkoniumdioxid, Magnesiumoxid, Zinkoxid oder deren Gemische, weiter bevorzugt Aluminiumoxid und Zirkoniumdioxid, eingesetzt.

Weitere Details bezüglich Katalysator 2 bzw. zu seiner Herstellung sind der DE-A 196 24 485 zu entnehmen, deren diesbezüglicher Inhalt durch Bezugnahme vollständig in die vorliegende Anmeldung einbezogen wird.

### KATALYSATOR 3

Der erfindungsgemäß verwendete Katalysator 3 kann dadurch hergestellt werden, dass das Trägermaterial in Form einen Metallgewebes oder einer Metallfolie nacheinander an der Luft erhitzt, abgekühlt und im Vakuum mit dem oben beschriebenen Aktivmetall oder der Kombination aus zwei oder mehr davon beschichtet wird, anschließend das beschichtete Trägermaterial geschnitten, verformt und letztendlich zu einem monolithischen Katalysatorelement verarbeitet wird. Dieser Katalysator und seine Herstellung sind bereits in der EP-A-0 564 830 und der US 4,686,202 ausführlich beschrieben, deren diesbezüglicher Inhalt vollumfänglich in den Kontext der vorliegenden Anmeldung einbezogen wird. Im folgenden sollen lediglich noch einmal die wesentlichen Grundzüge der Herstellung dieser Katalysators bzw. die bevorzugten Ausführungsformen desselben kurz diskutiert werden, wobei bezüglich der verwendeten Aktivmetalle das bezüglich der Katalysatoren 1 bis 2 Gesagte gilt.

Als metallische Trägermaterialien in Form von Metallfolien oder Metallgeweben eignen sich Edelstähle, wie beispielsweise die mit den Werkstoffnummern 1.4767, 1.4401, 2.4610, 1.4765, 1.4847, 1.4301 etc. besonders gut, da sie vor der Beschichtung mit Aktivkomponenten durch eine Temperung eine Aufrauung ihrer Oberfläche erhalten können. Dazu werden die metallischen Träger bei Temperaturen von 600 bis 1.100 °C, vorzugsweise 800 bis 1.000 °C 1 bis 20, vorzugsweise 1 bis 10 Stunden an der Luft erhitzt und wieder abgekühlt. Diese Vorbehandlung ist für die Aktivität des Katalysators entscheidend. Nach dieser Trägerbehandlung bei erhöhter Temperatur wird mit der Aktivkomponente beschichtet. Dazu wird der Träger im Vakuum bei einem Druck von 10⁻³ bis 10⁻⁵ mbar mittels einer Verdampfungseinrichtung, z.B. Elektronenstrahlverdampfung oder Sputtervorrichtung mit der Aktivkomponente gleichzeitig oder nacheinander in diskontinuierlicher oder kontinuierlicher Fahrweise beschichtet. Zur Formierung des Katalysators kann eine Temperung unter Inertgas oder Luft nachgeschaltet werden.

Dabei geht es bei der hier beschriebenen Herstellung von Katalysatorschichten darum, möglichst ungeordnete und gestörte polykristalline Schichten bzw. Cluster herzustellen. Deshalb sind normalerweise keine besonders guten Vakuumbedingungen erforderlich. Ferner kann man durch wechselweises Aufdampfen von Aktivkomponenten und strukturellen Promotoren die Aktivkomponenten sehr feinkristallin bzw. clusterförmig erzeugen.

Man kann hier den Katalysator systematisch aufbauen, beispielsweise in einer Bedampfungsanlage mit mehreren verschiedenen Verdampfungsquellen. So lässt sich beispielsweise zunächst eine Oxidschicht oder durch reaktives Verdampfen eine Haftschicht auf dem Träger aufbringen. Auf diese Grundschicht lassen sich Aktivkomponenten und Promotoren in mehreren Wechselschichten herstellen. Durch Einlass eines reaktiven Gases in den Rezipienten können Promotorschichten aus Oxiden oder anderen Verbindungen erzeugt werden. Auch Tempervorgänge können zwischengeschaltet werden.

Durch diese Art der Herstellung des Katalysatorgewebes bzw. der Katalysatorfolien besitzen die Aktivkomponenten eine so gute Haftfestigkeit, dass man sie nun schneiden, verformen und zu monolithischen Katalysatorelemente verarbeiten kann.

Einen sehr einfachen monolithischen Katalysator erhält man, wenn man das Katalysatorgewebe bzw. die Katalysatorfolie mittels einer Zahnradwalze verformt und glattes und gewelltes Gewebe bzw. Folie zusammenrollt zu einem zylinderförmigen Monolithen mit gleichartigen senkrechten Kanälen. Es lassen sich aber auch beliebige statische Mischer aus diesem Katalysatormaterial formen, da die Haftfestigkeit der Katalysatorschicht ausreichend hoch ist.

Die so erzeugten monolithischen Katalysatorelemente in Form von Mischelementen werden in einem Reaktor eingebaut und mit einer umzusetzenden Reaktionsflüssigkeit beaufschlagt.

### DIE VERFAHRENSFÜHRUNG

Im Rahmen der erfindungsgemäßen Verfahren wird die Hydrierung im allgemeinen bei einer Temperatur von ungefähr 50 bis ungefähr 140°C, vorzugsweise ungefähr 80 bis ungefähr 120°C durchgeführt. Die dabei verwendeten Drücke liegen in der Regel oberhalb von ungefähr 50 bar, bevorzugt ungefähr 80 bis ungefähr 300 bar, besonders bevorzugt ungefähr 100 bis ungefähr 160 bar.

Die erfindungsgemäßen Verfahren können entweder kontinuierlich oder diskontinuierlich durchgeführt werden, wobei die kontinuierliche Verfahrensführung bevorzugt ist. Bei der kontinuierlichen Verfahrensführung können die jeweiligen Katalysatoren als Festbettkatalysatoren oder in Suspension eingesetzt werden. Vorzugsweise werden die erfindungsgemäßen Katalysatoren als Festbettkontakte eingesetzt. In Suspensionsfahrweise werden die erfindungsgemäßen Verfahren vorzugsweise in einem Reaktor durchgeführt, der eine Vorrichtung umfasst, die Öffnungen oder Kanäle mit einem hydraulischen Durchmesser von 0,5 bis 20 mm, vorzugsweise 1 bis 10 mm und insbesondere 1 bis 3 mm aufweist, einer sogenannten Blasensäule, durchgeführt. Weitere Details bzgl. dieses speziellen Reaktors sind der DE-A 196 11 976 zu entnehmen, deren Inhalt vollumfänglich in den Kontext der vorliegenden Anmeldung aufgenommen wird.

Bei der kontinuierlichen Verfahrensführung beträgt die Menge des zur Hydrierung vorgesehenen Zuckers ungefähr 0,05 bis ungefähr 3 kg/l Katalysator pro Stunde, weiter bevorzugt ungefähr 0,1 bis ungefähr 1 kg/l Katalysator pro Stunde.

Als Hydriergase können beliebige Gase verwendet werden, die freien Wasserstoff enthalten und keine schädlichen Mengen an Katalysatorgiften, wie beispielsweise CO, aufweisen. Beispielsweise können Reformerabgase verwendet werden. Vorzugsweise wird reiner Wasserstoff als Hydriergas verwendet.

Die umzusetzenden Zucker werden in reiner Form als wässrige Lösungen eingesetzt. Vorzugsweise betragen die Konzentrationen des Zuckers ungefähr 15 bis ungefähr 70 Gew.-%, weiter bevorzugt ungefähr 30 bis ungefähr 55 Gew.-%, bezogen auf das Gesamtgewicht der wässrigen Lösung.

Der pH-Wert der wässrigen Lösung beträgt im allgemeinen ungefähr 3,5 bis ungefähr 10, vorzugsweise ungefähr 3,5 bis ungefähr 8. Die wässrigen Zuckerlösungen sind aufgrund geringfügiger Verunreinigungen durch Zuckersäuren im allgemeinen neutral oder schwach sauer. Sie können auf eine dem Fachmann bekannte Weise auf den gewünschten pH-Wert eingestellt werden. Prinzipiell kann die Hydrierung der Zucker auch ohne Veränderung des pH-Wertes durchgeführt werden, wobei eine spätere - ggf. aufwendige - Abtrennung der Substanzen, die zur pH-Wert-Einstellung eingesetzt werden, entfällt.

In den erfindungsgemäßen Verfahren ist der Umsatz der Zucker bei der Hydrierung nahezu vollständig. Es konnte maximal 0,1 Gew.-% des eingesetzten Zuckers in der Produktlösung nachgewiesen werden. Die Zuckeralkohole fallen in nahezu Epimeren-freier Form an, d.h. der Gehalt an Epimeren im Produkt beträgt im allgemeinen nicht mehr als 1 Gew.-%, vorzugsweise nicht mehr als 0,5 Gew.-%, und mit hoher Reinheit (> 99 %) an. Der Gehalt an Schwermetallen liegt im allgemeinen unter 10, vorzugsweise unter 5 und insbesondere unter 1 ppm.

Demgemäß betrifft die vorliegende Erfindung insbesondere die Verwendung der hierin beschriebenen Verfahren zur Herstellung von Zuckeralkoholen, die nahezu Epimeren-frei sind.

Die nach Abschluss der Hydrierung anfallenden, den/die gewünschten Zuckeralkohol(e) enthaltenden Produktlösungen können, falls dies erwünscht ist, nach üblichen Methoden, wie z.B. Sprühtrocknung, Gefriertrocknung, Trokenwalzen oder Verdampfen, aufkonzentriert werden.

Jedenfalls können sie ohne weitere Reinigungsstufe konzentriert oder unkonzentriert weiter verarbeitet werden.

Die so erhaltenen Zuckeralkohole, insbesondere Sorbitol, können als Lebensmittelzusatzstoff, insbesondere als Süßstoffe, Feuchtigkeitsstabilisatoren in Nahrungsmitteln sowie in Kosmetika und pharmazeutischen Produkten, als Grundstoffe für die Polyurethan-Herstellung, insbesondere zur Herstellung von harten Polyurethan-Schäumen verwendet werden.

Das erfindungsgemäß hergestellte Sorbitol kann insbesondere zur Synthese von Vitamin C verwendet werden.

Somit betrifft die vorliegende Erfindung ferner ein Verfahren zur Synthese von Vitamin C ausgehend von Sorbitol, das dadurch gekennzeichnet ist, dass das Ausgangsmaterial Sorbitol gemäß einem der hierin beschriebenen Verfahren erhalten wird.

Die erfindungsgemäßen Verfahren bzw. die Verwendung der hierin beschriebene Katalysatoren bei der Hydrierung von Zuckern zu Zuckeralkoholen bringt insbesondere dahingehend Vorteile, dass in den bei der Hydrierung erhaltenen Zuckeralkoholen keine nennenswerten Spuren von Metallen in kolloider oder ionischer Form, wie sie z.B. durch Zersetzung oder "Ausbluten" der Katalysatoren, insbesondere durch den chelatisierenden Effekt der Polyhydroxyverbindungen, entstehen können, nachzuweisen sind. Die Metallgehalte der Rohprodukte betragen im allgemein nicht mehr als 1 ppm. Somit entfällt die bei zahlreichen Verfahren des Standes der Technik notwendige Abtrennung der Schwermetalle aus den jeweiligen als Rohprodukt erhaltenen Zuckeralkoholen. Die Verfahren sind somit weitaus einfacher, kostengünstiger und ökologischer durchzuführen. Die als Rohprodukte anfallenden Zuckeralkohole erfüllen im allgemeinen die handelsüblichen Reinheitsbedingungen, etwa nach Deutschem Arzneibuch (DAB), Food Chemical Codex (FCC) oder Joint Experts Commitee on Food Additives (JECFA), und brauchen daher vor der Weiterverarbeitung z.B. im Lebensmittelbereich nicht weiter aufgereinigt zu werden.

Die vorliegende Erfindung soll nunmehr anhand eines Beispiels weiter erläutert werden.

### Beispiel

### Katalysatorherstellung

Ein meso-/makroporöser Aluminiumoxidträger in Form von 4 mm-Extrudaten, der eine BET-Oberfläche von 238 m²/g und ein Porenvolumen von 0,45 ml/g besaß, wurde mit einer wässrigen Ruthenium-(III)-nitrat-Lösung, die eine Konzentration von 0,8 Gew.-% aufwies, getränkt. 0,15 ml/g (ungefähr 33% des Gesamtporenvolumens) der Poren des Trägers besaßen einen Durchmesser im Bereich von 50 nm bis 10.000 nm und 0,30 ml/g (ungefähr 67% des Gesamtporenvolumens) der Poren des Trägers wiesen einen Porendurchmesser im Bereich von 2 bis 50 nm auf. Das während des Tränkens vom Träger aufgenommene Lösungsvolumen entsprach dabei in etwa dem Porenvolumen des verwendeten Trägers.

Anschließend wurde der mit der Ruthenium-(III)-nitrat-Lösung getränkte Träger bei 120°C getrocknet und bei 200°C im Wasserstrom aktiviert (reduziert). Der so hergestellte Katalysator enthielt 0,5 Gew.-% Ruthenium, bezogen auf das Gewicht des Katalysators.

### Hydrierung von Glucose

In einem 300 ml-Druckreaktor wurden 7g des Ru-Katalysators gemäß des Herstellungsbeispiels in einem Katalysator-Korbeinsatz vorgelegt und mit 150g (0,42 mol) einer 50 Gew.-%-igen Glucose-Lösung (pH-Wert 5,5) versetzt. Die Hydrierung wurde mit reinem Wasserstoff bei einem konstanten Druck von 150 bar und einer Temperatur von 100°C durchgeführt. Es wurde solange hydriert, bis kein Wasserstoff mehr aufgenommen wurde (10h). Der Reaktor wurde anschließend entspannt. Der Umsatz an Glucose betrug 99,95%. Die Ausbeute an Sorbitol lag bei 99,1%, die Ausbeute an Mannitol lag bei ca. 0,5%, jeweils bezogen auf die Gesamtmenge der eingesetzten Glucose. Der Ruthenim-Gehalt in den erhaltenen Zuckeralkoholen lag unter 1 ppm.

## Patentansprüche

1. Verfahren zur Hydrierung eines Zuckers oder eines Gemisches aus zwei oder mehr davon, das die folgende Stufe umfasst:
Inkontaktbringen des Zuckers oder des Gemisches aus zwei oder mehr davon mit Wasserstoff in Gegenwart eines Katalysators, wobei ein Zuckeralkohol oder ein Gemisch aus zwei oder mehr davon erhalten wird, **dadurch gekennzeichnet, dass** der Katalysator mindestens eine in situ auf einem Träger abgeschiedene, homogene Verbindung mindestens eines Metalls der VIII. Nebengruppe des Periodensystems alleine oder zusammen mit mindestens einem Metall der I. oder VII. Nebengruppe des Periodensystems umfasst.

2. Verfahren nach Anspruch 1, wobei der Zucker ausgewählt wird aus der Gruppe bestehend aus Glucose, Mannose, Fructose, Xylose, Lactose, Maltose und Gemischen aus zwei oder mehr davon.

3. Verfahren nach einem der Ansprüche 1 oder 2, **dadurch gekennzeichnet, dass** der Katalysator als Festbett vorliegt und das Verfahren kontinuierlich durchgeführt wird.

4. Verfahren zur Synthese von Vitamin C ausgehend von Sorbitol, das die folgende Stufe umfasst:
Herstellung von Sorbitol ausgehend von Glucose mittels eines Verfahrens gemäß einem der Ansprüche 1 bis 3.

5. Verwendung eines Verfahrens gemäß einem der Ansprüche 1 bis 4 zur Herstellung von Zuckeralkoholen mit einem Epimeren-Gehalt von weniger als 1 Gew.-%.
